(19) Europäisches Patentamt
European Patent Office
Office européen des brevets

(11) Veröffentlichungsnummer: **0 002 206**
**A1**

(12) # EUROPÄISCHE PATENTANMELDUNG

(21) Anmeldenummer: **78101377.6**

(22) Anmeldetag: **16.11.78**

(51) Int. Cl.[2]: **C 07 C 53/36**
C 07 C 51/58
//C07C53/32, C07C53/34,
C07C51/00, C07C69/63,
C07C67/30, C07C49/27,
C07C45/02, C07C61/18,
C07C121/66, C07C120/00,
C07C121/75, C07C45/00,
C07C27/02

(30) Priorität: **24.11.77 CH 14405/77**
**26.10.78 CH 11075/78**

(43) Veröffentlichungstag der Anmeldung:
**13.06.79 Patentblatt 79/12**

(84) Benannte Vertragsstaaten:
**BE CH DE FR GB NL**

(71) Anmelder: **CIBA-GEIGY AG**
**Patentabteilung Postfach**
**CH-4002 Basel(CH)**

(72) Erfinder: **Martin, Pierre, Dr.**
**Meisenweg 38**
**CH-4310 Rheinfelden(CH)**

(72) Erfinder: **Steiner, Eginhard, Dr.**
**Obere Hofackerstrasse 3**
**CH-4414 Füllinsdorf(CH)**

(54) **Halogenierte Buttersäurechloride, Verfahren zu deren Herstellung und ihre Verwendung als Zwischenprodukte zur Herstellung von Schädlingsbekämpfungsmitteln.**

(57) Halogenierte Buttersäurechloride der Formel

$$\begin{array}{ccccccccc} & & Cl & & & & Cl & & \\ X & - & C & - & CH_2 & - & C & - & COCl \\ & & Y & & & & Cl & & \end{array}$$

worin X Fluor und Y Wasserstoff oder Fluor oder X Chlor und Y Wasserstoff oder Chlor bedeuten, Verfahren zu deren Herstellung und ihre Verwendung als Zwischenproduckte zur Herstellung von Schädlingsbekämpfungsmitteln.

EP 0 002 206 A1

Croydon Printing Company Ltd.

5-11470/1+2/=/ZFO

Halogenierte Buttersäurechloride, Verfahren zu deren Herstellung und ihre Verwendung als Zwischenprodukte zur Herstellung von Schädlingsbekämpfungsmitteln

Die vorliegende Erfindung betrifft halogenierte Buttersäurechloride und ein Verfahren zu deren Herstellung. Die halogenierten Buttersäurechloride stellen Zwischenprodukte zur Herstellung von Schädlingsbekämpfungsmitteln dar.

Die halogenierten Buttersäurechloride entsprechen der Formel

$$X-\underset{Y}{\overset{Cl}{C}}-CH_2-\underset{Cl}{\overset{Cl}{C}}-COCl \qquad (I),$$

worin

X Fluor und Y Wasserstoff oder Fluor oder
X Chlor und Y Wasserstoff oder Chlor bedeuten.

Die Verbindungen der Formel I können dadurch hergestellt werden, dass man eine Verbindung der Formel

$$CCl_3-CO-Z \qquad (II)$$

in Gegenwart eines Katalysators und in Gegenwart eines organischen Lösungsmittels an eine Verbindung der Formel

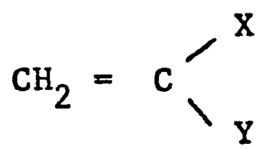

(III)

anlagert, wobei X und Y die unter Formel I angegebene Bedeutung haben und

Z -Cl, -OH oder -OAlkyl mit 1-4 C-Atomen darstellt,

und die Zwischenprodukte der Formel Ia

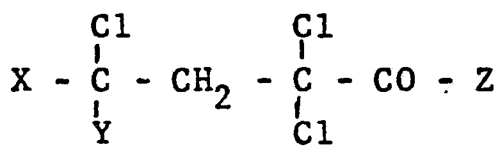

(Ia),

worin

Z -OH oder -OAlkyl mit 1-4 C-Atomen darstellt,

anschliessend in eine Verbindung der Formel I überführt.

Bevorzugt sind Verbindungen der Formel I, worin X und Y je Fluor und insbesondere solche, worin X und Y je Chlor bedeuten.

Die Ausgangsverbindungen der Formel II und III sind bekannt. Sie werden in mindestens stöchiometrischer Menge eingesetzt, bevorzugt verwendet man aber einen Ueberschuss an Verbindung der Formel III, beispielsweise einen etwa 0,5 bis 2-fachen molaren Ueberschuss, wobei die Verbindung der Formel II gegebenenfalls auch als Lösungsmittel dienen kann.

Ein Zwischenprodukt der Formel Ia (X = Cl, Y = H, Z = OAethyl) ist bekannt [vgl.z.B. Chemical Abstracts, 73, 24833g (1970)].

Als Katalysatoren können im erfindungsgemässen Verfahren an sich bekannte Verbindungen eingesetzt werden, wie Metalle der Hauptgruppe VIII und der Nebengruppen VIa, VIIa und Ib des Periodischen Systems (gemäss Lehrbuch der anorgan.Chemie, Holleman-Wiberg, U.de Gruyter & Co.,Berlin), z.B. Eisen,Kobalt, Nickel, Ruthenium, Rhodium, Palladium, Chrom, Molybdän, Mangan und Kupfer. Diese Metalle können in elementarer Form oder in Form von Verbindungen eingesetzt werden. Geeignete derartige Verbindungen sind beispielsweise Oxide, Halogenide, Sulfate, Sulfite, Sulfide, Nitrate, Acetate, Citrate, Carbonate, Cyanide und Rhodanide, sowie Komplexe mit Liganden, wie Phosphinen, Benzoin, Benzoyl- und Acetylacetonaten, Phosphiten, Nitrilen, Isonitrilen und Kohlenmonoxid.
Als Beispiele seien genannt: Kupfer(II)oxid, Eisen(II)oxid; Cu(I)-, Cu(II)-, Fe(II)- und Fe(III)bromide und vor allem -chloride sowie die Chloride des Rutheniums, Rhodiums, Palladiums, Kobalts und Nickels; Cu(II)sulfat, Fe(II)- und Fe(III)sulfat; Cu(II)nitrat und Eisen(III)nitrat; Mangan(III)acetat, Kupfer(II)-acetat, Kupfer(II)stearat; Eisen(III)citrat; Cu(I)cyanid; Ruthenium(II)dichloro-tris-triphenylphosphin, Rhodium-tris-(triphenylphosphin)chlorid; Chrom- und Nickelacetylacetonat, Kupfer(II)acetylacetonat, Eisen(III)acetylacetonat, Kobalt(II)- und Kobalt(III)acetylacetonat, Mangan(II)acetylacetonat, Kupfer(II)benzoylacetonat; Eisencarbonyl-cyclopentadienyl-komplex; Molybdäncarbonylcyclopentadienylkomplex, Chrom-tricarbonylarylkomplexe, Ruthenium(II)acetatokomplex, Chrom- und Molybdänhexacarbonyl, Nickeltetracarbonyl, Eisenpentacarbonyl, Kobalt- und Mangancarbonyl.

Es können auch Gemische der genannten Metalle mit Metallverbindungen und/oder anderen Zusätzen verwendet werden, wie Kupferpulver in Kombination mit einer der vorerwähnten Kupferverbindungen; Gemische von Kupferpulver mit Lithiumhalogeniden, wie Lithiumchlorid, oder mit Isocyaniden,

wie tert-Butylisocyanid; Gemische von Eisenpulver mit Eisen(III)chlorid, gegebenenfalls unter Zusatz von Kohlenmonoxid; Gemische von Eisen(III)chlorid und Benzoin; Gemische von Eisen(II)- oder Eisen(III)chlorid und Trialkylphosphiten; Gemische von Eisenpentacarbonyl und Jod.

Bevorzugt sind Eisen(II)- und Eisen(III)salze und -komplexe sowie Eisenpulver, vor allem jedoch Kupferpulver, Kupfer(I)- und Kupfer(II)salze und -komplexe, wie Cu(I)chlorid, Cu(II)-chlorid, Cu(I)bromid, Cu(II)bromid, Cu(II)acetylacetonat, Cu(II)benzoylacetonat, Cu(II)sulfat, Cu(II)nitrat und Cu(I)-cyanid.

Ganz besonders bevorzugt sind Kupferpulver, Kupfer(I)- und Kupfer(II)chlorid bzw. -bromid sowie deren Gemische.

Die Katalysatoren werden im allgemeinen in Mengen von etwa 0,01 bis 10 Mol.%, bevorzugt 0,1 bis 5 Mol.%, bezogen auf die Verbindung der Formel III, verwendet.

Die erfindungsgemässe Umsetzung wird in einem organischen Lösungsmittel vorgenommen. Geeignete organische Lösungsmittel sind solche, in denen die Katalysatoren ausreichend löslich sind oder die mit den Katalysatoren Komplexe bilden können, die aber gegenüber den Ausgangsverbindungen der Formel II und III inert sind. Beispiele solcher Lösungsmittel sind Alkylnitrile, insbesondere solche mit 2-5 C-Atomen, wie Acetonitril, Propionitril und Butyronitril; 3-Alkoxypropionitrile mit 1 oder 2 C-Atomen im Alkoxyteil, wie 3-Methoxypropionitril und 3-Aethoxypropionitril; aromatische Nitrile, vor allem Benzonitril; aliphatische Ketone mit bevorzugt insgesamt 3-8 C-Atomen, wie Aceton, Diäthylketon, Methyl-isopropylketon, Di-isopropylketon, Methyl-tert-butylketon; Alkyl- und Alkoxyalkylester von aliphatischen Monocarbonsäuren mit insgesamt 2-6 C-Atomen, wie Ameisensäuremethyl- und -äthylester, Essigsäuremethyl-, -äthyl-, -n-butyl- und -isobutylester sowie 1-Acetoxy-2-methoxyäthan; cyclische Aether, wie Tetrahydrofuran, Tetrahydropyran und Dioxan; Dialkyläther mit je 1-4 C-Atomen in den Alkylteilen, wie Diäthyläther, Di-n-propyläther und Di-isopropyläther; N,N-Dialkylamide von aliphatischen Monocarbonsäuren mit 1-3 C-Atomen im Säureteil, wie N,N-Dimethylformamid, N,N-Dimethylacetamid, N,N-Diäthylacetamid und N,N-Dimethyl-methoxy-acetamid; Aethylenglykol- und Diäthylenglykoldialkyläther mit je 1-4 C-Atomen in den Alkylteilen, wie Aethylenglykoldimethyl-, -diäthyl- und -di-n-butyläther, Diäthylenglykoldiäthyl- und -di-n-butyläther; Hexamethylphosphorsäuretriamid.

Bevorzugte Lösungsmittel sind Alkylnitrile mit 2-5 C-Atomen und 3-Alkoxypropionitrile mit 1 oder 2 C-Atomen im Alkoxyteil, insbesondere Acetonitril und 3-Methoxypropionitril

Die Reaktionstemperatur ist im allgemeinen nicht kritisch und kann innerhalb weiter Grenzen variieren. Bevorzugt liegen die Reaktionstemperaturen zwischen etwa 60 und 200°C, insbesondere zwischen etwa 80 und 170°C. Die Reaktion kann unter Druck oder drucklos durchgeführt werden.

Als Verbindung der Formel III verwendet man bevorzugt Trichloressigsäurechlorid oder einen Trichloressigsäurealkylester mit 1 oder 2 C-Atomen im Alkylteil.

Zwischenprodukte der Formel Ia, worin Z = -OH, können auf an sich bekannte Weise durch Behandeln mit geeigneten Chlorierungsmitteln, wie Phosphortrichlorid, Phosphorpentachlorid, Phosphoroxychlorid, Oxalylchlorid, Phosgen, Thionylchlorid, Benzoylchlorid oder Phthaloylchlorid, in Verbindungen der Formel I übergeführt werden.

Verwendet man als Verbindung der Formel III einen definitionsgemässen Trichloressigsäurealkylester, so kann das erhaltene Zwischenprodukt der Formel Ia mit Z = -OAlkyl mit 1-4 C-Atomen auf an sich bekannte Weise in Gegenwart starker Säuren, wie konzentrierte Salzsäure, zur entsprechenden Buttersäure hydrolysiert werden, worauf man diese, wie oben erwähnt, durch Behandeln mit geeigneten Chlorierungsmitteln in Verbindungen der Formel I überführt.

Nach Beendigung der Umsetzung können die Verbindungen der Formel I auf konventionelle Weise isoliert und allenfalls gereinigt werden, beispielsweise mittels Destillation.

Die Verbindungen der Formel I stellen wertvolle Zwischenprodukte zur Herstellung von Schädlingsbekämpfungsmitteln dar. Sie können auch nach einer neuen, eigenartigen Synthese in pyrethroidartige Schädlingsbekämpfungsmittel oder Vorläufer davon übergeführt werden. Derartige Verbindungen, beispielsweise solche der Formel

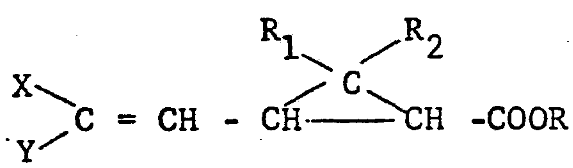
(IV),

worin X und Y die unter Formel I angegebene Bedeutung haben, eines von $R_1$ und $R_2$ Methyl und das andere Wasserstoff oder Methyl oder $R_1$ und $R_2$ zusammen Alkylen mit 2-4 C-Atomen bebedeuten,

R Wasserstoff, Alkyl mit 1-4 C-Atomen oder eine Gruppierung 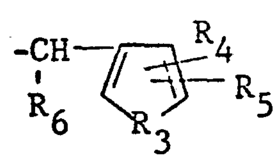 darstellt, wobei

$R_3$ -O-, -S- oder -CH=CH-,

$R_4$ Wasserstoff, Alkyl mit 1-4 C-Atomen, Benzyl, Phenoxy oder Phenylmercapto,

$R_5$ Wasserstoff oder Alkyl mit 1-4 C-Atomen und

$R_6$ Wasserstoff oder Aethinyl oder, wenn eines von $R_1$ und $R_2$ Methyl und das andere Wasserstoff oder Methyl, $R_3$ -CH=CH-, $R_4$ Phenoxy und $R_5$ Wasserstoff bedeuten, auch Alkyl mit 1-5 C-Atomen darstellen,

können dadurch erhalten werden, dass man eine Verbindung der Formel I in Gegenwart eines Dehalogenierungsmittels mit einer Verbindung der Formel

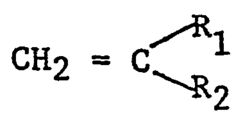

(V)

zu einer Verbindung der Formel

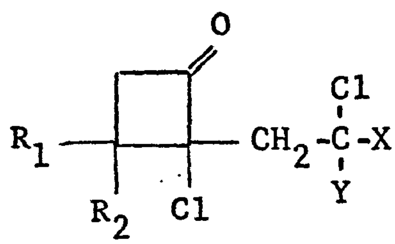

(VI)

umsetzt, die Verbindungen der Formel VI in Gegenwart eines Katalysators in eine Verbindung der Formel

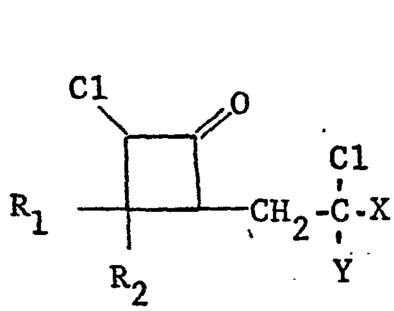

(VII)

umlagert und die Verbindung der Formel VII in Gegenwart einer Base in eine Verbindung der Formel IV überführt.

Bei der Umsetzung der Verbindungen der Formel I mit dem Dehalogenierungsmittel wird intermediär ein Keten der Formel

$$\begin{array}{ccccccc} & Cl & & & Cl & & \\ & | & & & | & & \\ X - & C & - CH_2 - & & C & = C = O & \\ & | & & & & & \\ & Y & & & & & \end{array} \qquad (VIII)$$

gebildet, das mit den Verbindungen der Formel V zu Verbindungen der Formel VI cycloaddiert. Als Dehalogenierungsmittel können an sich bekannte halogenabspaltende Mittel eingesetzt werden. Insbesondere kommen gegebenenfalls durch HCl, Kupfer oder Silber aktiviertes Zink, Zinn, Raney-Nickel, Raney-Kobalt, Quecksilber, Silber und Magnesium in Betracht. Bevorzugtes Dehalogenierungsmittel ist Zink, das in an sich beliebiger Form, z.B. als Pulver, in Form von Zinkwolle oder Zinkspänen, eingesetzt werden kann.

Die genannte Umsetzung wird zweckmässig in einem inerten organischen Lösungsmittel vorgenommen, wie Aethern der vorerwähnten Art, besonders Dialkyläthern mit je 1-4 C-Atomen in den Alkylteilen, aliphatischen Ketonen, besonders solchen, die in α-Stellung verzweigt sind, wie Diisopropylketon und Methyl-tert-butylketon, oder Alkyl- und Alkoxyalkylestern von aliphatischen Monocarbonsäuren mit insgesamt 2-6 C-Atomen, wie Ameisensäuremethyl- und -äthylester, Essigsäuremethyl-, -äthyl-, n-butyl- und -isobutylester sowie 1-Acetoxy-2-methoxyäthan.

Im allgemeinen ist es auch zweckmässig, die Umsetzung in Gegenwart von schwachen Lewis-Säuren, wie Ammoniumchlorid, Natrium-, Kalium- und Quecksilberjodid, Zinkchlorid, $MePOCl_2$, $POCl_3$ oder auch elementarem Jod durchzuführen. Es können auch Gemische solcher Lewis-Säuren eingesetzt werden.

Die Reaktionstemperaturen liegen mit Vorteil zwischen etwa 15 und 80°C, insbesondere zwischen etwa 25 und 65°C.

Die Olefine der Formel V werden in mindestens äquimolarer Menge, bezogen auf das Säurechlorid der Formel I bzw. das in situ gebildete Keten der Formel VIII, verwendet. Im allgemeinen wird aber mit Vorteil ein Ueberschuss an Olefin der Formel V verwendet.

Die Ketene der Formel VIII und die Verbindungen der Formel VI und VII sind neu.

Als Katalysatoren für die Umlagerung der Verbindungen der Formel VI in Verbindungen der Formel VII können beispielsweise Säuren, Basen oder quaternäre Ammoniumhalogenide verwendet werden. Die Umlagerung kann in der Schmelze oder in einem geeigneten inerten organischen Lösungsmittel vorgenommen werden.

Als Basen für die Ueberführung der Verbindungen der Formel VII in Verbindungen der Formel IV können z.B. Alkalimetall- und Erdalkalimetallhydroxide oder -alkoholate eingesetzt werden.

Nach dieser neuen erfinderischen Synthese können über die neuen Zwischenprodukte der Formeln VI und VII Verbindungen der Formel IV im Vergleich zu vorbekannten Verfahren [vgl.z.B. Farkas et al.,Coll.Czech.Chem.Comm.24,2230(1959) und Chem.Listy, 52,688(1958); britische Patentschrift 1.285.350; belgische Patentschrift 850.402; deutsche Offenlegungsschriften 2.439.177, 2.539.895, 2.544.150, 2.547.510, 2.552.615, 2.554.380, 2.605.398, 2.615.159, 2.615.160, 2.616.528, 2.621.830, 2.621.832, 2.621.833, 2.621.835, 2.623.777, 2.630.981 und 2.639.777; US Patentschrift 3.961.070 und japanische Offenlegungsschriften 69872/76 und 47966/76] auf besonders einfache und wirtschaftliche Weise, in guten bis sehr guten Ausbeuten und mit hohen Anteilen der erwünschten, bei gewissen Anwendungen besonders wirksamen cis-Form hergestellt werden. Im Gegensatz zu vorbekannten Verfahren sind die Ausgangsprodukte leicht

zugänglich, die verwendeten Reagenzien sind vergleichsweise billig und ökologisch günstig, sämtliche Reaktionsschritte können unter relativ milden Bedingungen und ohne grossen apparativen Aufwand und sicherheitstechnische Massnahmen durchgeführt werden. Die Zwischenprodukte der Formeln VI und VII weisen die für ihre Weiterverwendung erforderliche Oxidationsstufe auf, so dass weder Oxidationen noch Reduktionen erforderlich sind. Die einzelnen Verfahrensschritte können auch ohne Isolierung des jeweiligen Zwischenprodukts unmittelbar nacheinander durchgeführt werden, so dass sich diese Synthese sehr gut für die grosstechnische Herstellung von Verbindungen der Formel IV eignet.

Verbindungen der Formel IV, worin R Wasserstoff oder Alkyl mit 1-4 C-Atomen darstellt, können auf an sich bekannte Weise in Wirkstoffe der Formel IV mit R ≠ H oder Alkyl mit 1-4 C-Atomen übergeführt werden, oder auch in Verbindungen der Formel IV, worin $R_6$ zusätzlich -CN bedeutet, z.B. durch Umsetzung mit entsprechenden Halogeniden oder Alkoholen, gegebenenfalls unter vorheriger Ueberführung in das Säurechlorid, oder durch Umesterung [vgl. z.B. die deutschen Offenlegungsschriften 2.553.991 und 2.614.648].

Verbindungen der Formel IV mit R ≠ H oder Alkyl mit 1-4 C-Atomen eignen sich zur Bekämpfung verschiedenartigster tierischer und pflanzlicher Schädlinge, besonders als Insektizide. Die Eigenschaften, Anwendungsgebiete und Anwendungsformen dieser Wirkstoffe sind in der Literatur beschrieben [vgl. z.B. Nature, 246,169-70(1973); Nature, 248,710-11(1974); Proceedings 7th British Insecticide and Fungicide Conference,721-728 (1973); Proceedings 8th British Insecticide and Fungicide Conference, 373-78(1975); J.Agr.Food Chem.,23,115(1975); US Patentschrift 3.961.070; deutsche Offenlegungsschriften 2.553.991, 2.439.177, 2.326.077 und 2.614.648].

0002206

Beispiel 1

a) 242,5 g (2,5 Mol) 1,1-Dichloräthylen,356 g (2 Mol) Trichloressigsäuremethylester, 12,8 g Kupfer(I)chlorid und 800 ml Acetonitril werden während 24 Stunden bei 115°C gehalten. Das Reaktionsgemisch wird klarfiltriert und eingedampft. Der Rückstand wird destilliert. Man erhält 293,7 g 2,2,4,4,4-Pentachlorbuttersäuremethylester; Sdp. 118-122°C/ 15 mm Hg.

IR-Spektrum (Film) in $cm^{-1}$: 1755 und 1772 (CO).

NMR-Spektrum (100 MHz, $CDCl_3$) in ppm: 4,08 (s, $CH_2$, $^1J_{(C,H)}$= 139 Hz); 3,93 (s, 3H, $CH_3$, $^1J_{(C,H)}$= 148 Hz).

Elementaranalyse für $C_5H_5Cl_5O_2$ (Molgewicht 274,36):

| | | | |
|---|---|---|---|
| berechnet | C 21,89% | H 1,84% | Cl 64,61% |
| gefunden | C 22,0% | H 1,8% | Cl 64,3%. |

Als Nebenprodukt destilliert bei 150°C/15 mm Hg 156,7 g Bis-(2',2',2'-trichloräthyl)-chloressigsäuremethylester; Smp. 65-67°C (aus n-Hexan umkristallisiert); NMR (100 MHz,$CDCl_3$) in ppm: 3,86 (s, 3H, $CH_3$); 3,77 (AB-System, 4H, $CH_2$).

b) 145,4 g (1,5 Mol) 1,1-Dichloräthylen,712 g (4 Mol) Trichloressigsäuremethylester, 12,8 g Kupfer(I)chlorid und 800 ml Acetonitril werden wie unter a) beschrieben zur Reaktion gebracht. Nach gleicher Aufarbeitung erhält man 289 g 2,2,4,4,4-Pentachlorbuttersäuremethylester. Dabei wird praktisch kein Nebenprodukt der unter a) beschriebenen Art gebildet.

Beispiel 2

382,9 g (2 Mol) Trichloressigsäureäthylester, 242,8 g (2,5 Mol) 1,1-Dichloräthylen,400 ml Acetonitril und 6,0 g Kupfer(I)chlorid werden während 4 Stunden auf 140°C erhitzt. Das Reaktionsgemisch wird klarfiltriert und destilliert. Man erhält 209,9 g 2,2,4,4,4-Pentachlorbuttersäureäthylester; Sdp. 140-145°C/20 mm Hg.

IR-Spektrum ($CHCl_3$) in $cm^{-1}$: 1770(CO).

NMR-Spektrum (60 MHzm $CDCl_3$) in ppm: 4,1 (s, 2H, $CH_2$); 4,30 (q, 2H, $CH_2$):, 1,40 (t, 3H, $CH_3$).

Elementaranalyse für $C_6H_7Cl_5O_2$ (Molgewicht 288,40):
berechnet C 24,99% H 2,45% Cl 61,47%
gefunden C 24,9% H 2,4% Cl 61,2%.

Beispiel 3

a) 145,4 g (1,5 Mol) 1,1-Dichloräthylen, 182 g (1 Mol) Trichloressigsäurechlorid, 3,0 g Kupfer(I)chlorid und 200 ml Acetonitril werden während 4 Stunden bei 140°C gehalten. Die Reaktionslösung wird klarfiltriert und eingedampft. Der Rückstand wird destilliert. Die Fraktion von 88-102°C/ 10 mm Hg wird redestilliert. Man erhält 115,7 g 2,2,4,4,4-Pentachlorbuttersäurechlorid; Sdp. 95-96°C/ 10 mm Hg.

IR-Spektrum ($CHCl_3$) in $cm^{-1}$: 1785 (CO).

NMR-Spektrum (60 MHz, $CDCl_3$) in ppm: 4,10 (s, $CH_2$).

Elementaranalyse für $C_4H_2Cl_6O$ (Molgewicht 278,80):
berechnet C 17,23% H 0,72% Cl 76,30%
gefunden C 17,8% H 1,0% Cl 75,5%.

b) 137 g (0,5 Mol) des gemäss Beispiel 1 erhaltenen 2,2,4,4,4-Pentachlorbuttersäuremethylesters werden mit 700 ml konzentrierter Salzsäure während 40 Stunden gekocht. Nach dem Erkalten wird mit Toluol extrahiert. Die Extrakte werden mit verdünnter Natronlauge extrahiert. Die alkalische, wässrige Phase wird mit Diäthyläther gewaschen, mit Salzsäure angesäuert und mit Diäthyläther extrahiert. Dieser Extrakt wird mit Wasser gewaschen, über Magnesiumsulfat getrocknet und eingedampft. Der Rückstand wird mit 200 ml Thionylchlorid während 2 Stunden bei 70°C gehalten. Das dunkle Reaktionsgemisch wird zur Trockne eingedampft. Der Rückstand wird destilliert. Man erhält 102,9 g 2,2,4,4,4-Pentachlorbuttersäurechlorid; Sdp. 100-102°C/14 mm Hg. Die spektroskopischen Daten des erhaltenen Säurechlorids sind mit denjenigen des gemäss a) hergestellten Produkts identisch.

c) Auf analoge Weise wie unter b) beschrieben kann der gemäss Beispiel 2 erhaltene 2,2,4,4,4-Pentachlorbuttersäureäthylester in das entsprechende Säurechlorid übergeführt werden.

Im folgenden wird die Ueberführung des 2,2,4,4,4-Pentachlorbuttersäurechlorids in je zwei Verbindungen der Formeln VI, VII und IV bzw. beschrieben:

A(1) 2-Chlor-2-(2',2',2'-trichloräthyl)-3,3-dimethylcyclobutanon

150 ml Diisopropylketon werden bei Raumtemperatur (20-25°C) mit Isobutylen gesättigt. Dazu gibt man 26,1 g Zinkspäne, 0,54 g Ammoniumchlorid und 0,17 g Kaliumiodid. Während 1 Stunde wird bei 30°C und unter Einleiten eines schwachen Stroms von Isobutylen eine Lösung von 27,9 g (0,1 Mol) 2,2,4,4,4-Pentachlorbuttersäurechlorid in 50 ml Diisopropylketon zugetropft. Anschliessend wird während 4,5 Stunden bei 30°C weitergerührt. Das Reaktionsgemisch wird klarfiltriert, mit verdünnter Salzsäure und Wasser gewaschen, über Magnesiumsulfat getrocknet und eingedampft. Der Rückstand wird aus n-Hexan kristallisiert. Man erhält 13,7 g (52% d.Th.) 2-Chlor-2-(2',2',2'-trichloräthyl)-3,3-dimethylcyclobutanon; Smp. 73-75°C.

IR-Spektrum ($CHCl_3$) in $cm^{-1}$: 1805 (CO).

$^1$H-NMR-Spektrum (100 MHz, $CDCl_3$) in ppm: 1,42 und 1,45 (je 1s, 6H, je 1 $CH_3$); 2,91-3,28 (m, 2H, $CH_2$); 3,37-3,76 (m, 2H, $CH_2$).

$^{13}$C-NMR-Spektrum ($CDCl_3$) in ppm: 196 (s, CO); 95,3 (s, $CH_3$); 80,8 (s, C-2); 57,0 (t, $CH_2$); 56,4 (t, $CH_2$); 37,9 (s, C-3); 25,1 (q, $CH_3$); 23,8 (q, $CH_3$).

Elementaranalyse für $C_8H_{10}Cl_4O$ (Molgewicht 263,98):

| | | | | |
|---|---|---|---|---|
| berechnet | C 36,40% | H 3,82% | O 6,02% | Cl 53,72% |
| gefunden | C 36,4% | H 3,9% | O 6,2% | Cl 53,5%. |

A (2) 2-(2',2',2'-Trichloräthyl)-3-3-dimethyl-4-chlorcyclobutanon

132 g (0,5 Mol) 2-Chlor-2-(2'-2',2'-trichloräthyl)-3,3-dimethylcyclobutan-1-on werden in 700 ml Toluol gelöst, mit 1 ml Triäthylamin versetzt und unter Rückfluss gekocht. Nach 13 Stunden Reaktionszeit wird erneut 1 ml Triäthylamin zugegeben und 7 Stunden weitergekocht. Nach dem Erkalten wird das Reaktionsgemisch zuerst mit verdünnter Salzsäure und dann mit Wasser gewaschen, getrocknet und eingedampft. Der erstarrte, dünnschichtchromatographisch einheitliche Rückstand (124 g; 94 % d.Th.) wird aus n-Hexan kristallisiert. Man erhält 105,8 g 2-(2',2',2'-Trichloräthyl)-3,3-dimethyl-4-chlorcyclobutan-1-on; Smp. 56-57°C.

IR-Spektrum ($CHCl_3$) in $cm^{-1}$: 1800 (CO).

$^1$H-NMR-Spektrum (100 MHz, $CDCl_3$) in ppm: 4,77 (d, J=2 Hz, 1H, H an C-4); 3,47 (m, 1H, H an C-2); 2,73-3,26 (m, 2H, $CH_2$); 2,63 (s, 3H, $CH_3$); 1,14 (s, 3H, $CH_3$).

$^{13}$C-NMR-Spektrum ($CDCl_3$) in ppm: 197,0 (s, CO); 97,8 (s, $CCl_3$); 69,4 (d, C-4); 60,6 (d, C-2); 49,5 (t, $\underline{C}H_2$-$CCl_3$); 36,8 (s, C-3); 27,4 (q, $CH_3$); 18,6 (q, $CH_3$).

Elementaranalyse für $C_8H_{10}Cl_4O$ (Molgewicht 263,98):

| | | | | |
|---|---|---|---|---|
| berechnet | C 36,40% | H 3,82% | O 6,02% | Cl 53,72% |
| gefunden | C 36,6% | H 3,8% | O 6,2% | Cl 53,6%. |

A(3) 2-(2',2'-Dichlorvinyl)-3,3-dimethylcyclopropancarbonsäure

13,2 g (0,05 Mol) 2-(2',2',2'-Trichloräthyl)-3,3-dimethyl-4-chlorcyclobutanon werden mit 150 ml 10%iger Natronlauge versetzt und intensiv gerührt. Nach 5 Minuten hat sich eine klare Lösung gebildet, die während 1 Stunde auf 100°C (Badtemperatur) erwärmt wird. Die Reaktionslösung wird mit Diäthyläther gewaschen, unter Kühlung mit konzentrierter Salzsäure angesäuert und mit Diäthyläther extrahiert. Die Aetherphase wird mit Wasser gewaschen, über Magnesiumsulfat getrocknet und eingedampft. Der feste Rückstand (10,35) setzt sich gemäss NMR-Spektrum aus 80 Gew.-% cis- und 20 Gew.-% trans-2-(2',2'-Dichlorvinyl)-3,3-dimethyl-cyclopropan-1-carbonsäure zusammen. Kristallisation aus n-Hexan liefert reine cis-Säure; Smp. 85-87°C.

IR-Spektrum ($CHCl_3$) in $cm^{-1}$: 1710 (CO), 1625 (C=C).

NMR-Spektrum (100 MHz, $CDCl_3/D_2O$) in ppm: 1,30 (s, 6H, 2 mal $CH_3$); 1,85 (d, J=8,5 Hz, 1H, HC-1); 2,02-2,19 (m, 1H, HC-2); 6,17 (d, J=8 Hz, 1H, CH=$CCl_2$).

A(4) cis-2-(2',2'-Dichlorvinyl)-3,3-dimethylcyclopropancarbonsäure-α-cyan-m-phenoxybenzylester

10 g (0,047 Mol) cis-2-(2',2'-Dichlorvinyl)-3,3-dimethylcyclopropancarbonsäure werden in 100 ml Benzol mit 12,1 ml (0,141 Mol) Oxalylchlorid 24 Stunden bei Raumtemperatur gerührt. Nach dem Eindampfen der Reaktionslösung wird der braune Rückstand unter vermindertem Druck destilliert. Man erhält 9,1 g einer klaren Flüssigkeit; Sdp. 50°C/0,04 mm Hg. 3,0 g dieser klaren Flüssigkeit werden in 30 ml Toluol gelöst und mit 2 ml Pyridin versetzt. Bei Raumtemperatur werden dazu 2,9 g α-Cyano-m-phenoxybenzylalkohol in 20 ml Toluol getropft, und das Reaktionsgemisch wird anschliessend während 16 Stunden bei Raumtemperatur weitergerührt. Das Reaktionsgemisch wird zuerst mit Wasser, dann mit gesättigter Natriumhydrogencarbonatlösung und anschliessend mit Sole gewaschen, über Magnesiumsulfat getrocknet und eingedampft. Der Rückstand wird über Kieselgel chromatographiert (Eluierung mit Diäthyläther/n-Hexan 1:2). Man erhält reinen cis-2-(2',2'-Dichlorvinyl)-3,3-dimethylcyclopropancarbonsäure-α-cyan-m-phenoxybenzylester als Diastereoisomerengemisch.

NMR-Spektrum (60 MHz, $CDCl_3$) in ppm: 1,20-1,43 (m, 6H, $CH_3$); 1,67-2,35 (m, 2H, 2x CH); 6,25 (d, J=9Hz, 1H, C<u>H</u>-$CCl_2$); 6,40 und 6,45 (je 1s, je 0,5H, C<u>H</u>-CN); 6,98-7,65 (m,9H).

Beispiel 4

a) 181,8 g (1 Mol) Trichloressigsäurechlorid, 3 g (Kupfer(I) chlorid und 200 ml Acetonitril werden in einem Tantalautoklaven vorgelegt. Nach dem Aufpressen von 93,7 g (1,5 Mol) Vinylchlorid wird der Autoklav 4 Stunden auf 140°C erhitzt. Das Reaktionsgemisch wird dann destilliert. Man erhält 2,2,4,4-Tetrachlorbuttersäurechlorid als farblose Flüssigkeit; Sdp. 100-105°C/20 mm Hg.

IR-Spektrum ($CHCl_3$) in $cm^{-1}$: 1790 (CO).

NMR-Spektrum (60 MHz, $CDCl_3$) in ppm: 3,60 (d, 2H, $CH_2$); 6,06 (t, 1H, CH).

Elementaranalyse für $C_4H_3Cl_5O$ (Molgewicht 244,40):

| | | | |
|---|---|---|---|
| berechnet | C 19,67% | H 1,24% | Cl 72,56% |
| gefunden | C 20,0% | H 1,3% | Cl 71,5% |

b) 191,4 g (1 Mol) Trichloressigsäureäthylester, 3 g Kupfer(I) chlorid und 200 ml Acetonitril werden in einem Tantalautoklaven vorgelegt. Nach dem Aufpressen von 93,7 g (1,5 Mol) Vinylchlorid wird der Autoklav während 4 Stunden auf 140°C erhitzt. Das Reaktionsgemisch wird anschliessend destilliert. Man erhält 208,5 g (82% d.Th.) 2,2,4,4-Tetrachlorbuttersäureäthylester; Sdp. 127-130°C/ 20 mm Hg.

IR-Spektrum ($CHCl_3$) in $cm^{-1}$: 1770 (CO).

NMR-Spektrum (60 MHz, $CDCl_3$) in ppm: 1,41 (t, 3H, CH3); 3,48 (d, 2H, $CH_2$); 4,36 (q, 2H, $CH_2$); 6,01 (t, 1H, CH).

Elementaranalyse für $C_6H_8Cl_4O_2$ (Molgewicht 237,95):

| | | | |
|---|---|---|---|
| berechnet | C 28,38% | H 3,18% | Cl 55,85% |
| gefunden | C 28,7% | H 3,5% | Cl 54,8% |

c) 50,8 g (0,2 Mol) 2,2,4,4-Tetrachlorbuttersäureäthylester und 200 ml konzentrierte Salzsäure werden während 72 Stunden am Rückfluss erhitzt. Nach dem Erkalten wird das Reaktionsgemisch mit Toluol ausgezogen. Der Extrakt wird mit Wasser gewaschen, über Magnesiumsulfat getrocknet und eingedampft.

Der Rückstand wird destilliert. Man erhält 40,2 g (86% d.Th.) 2,2,4,4-Tetrachlorbuttersäure; Sdp. 155-160°C/18 mm Hg.

IR-Spektrum ($CHCl_3$) in $cm^{-1}$: 1750 (CO).

NMR-Spektrum (60 MHz, $CDCl_3$) in ppm: 3,50 (d, 2H, $CH_2$); 6,01 (t, 1H, CH); 9,9 (s, 1H, OH).

Elementaranalyse für $C_4H_4Cl_4O_2$ (Molgewicht 225,89):

| | | | | |
|---|---|---|---|---|
| berechnet | C | 21,27% | H 1,78% | Cl 62,78% |
| gefunden | C | 21,9% | H 1,9% | Cl 61,9%. |

d) 45,18 g (0,2 Mol) 2,2,4,4-Tetrachlorbuttersäure und 100 ml Thionylchlorid werden während 2 Stunden auf 70°C erhitzt. Nach dem Erkalten wird das Reaktionsgemisch eingedampft. Die Destillation unter vermindertem Druck liefert 2,2,4,4-Tetrachlorbuttersäurechlorid als farblose Flüssigkeit; Sdp. 101-104°C/20 mm Hg.
Die spektroskopischen Daten des erhaltenen Säurechlorids sind identisch mit denjenigen des gemäss a) hergestellen 2,2,4,4-Tetrachlorbuttersäurechlorids.

Im folgenden wird die Aufarbeitung zu Verbindungen der Formel VI, VII und IV beschrieben:
500 ml Diisopropylketon werden bei Raumtemperatur mit Isobutylen gesättigt. Dazu gibt man 100 g Zinkspäne, 2,2g Ammoniumchlorid und 0,7g Kaliumiodid. Während 2,5 Stunden werden bei Raumtemperatur und unter Einleiten eines geringen Stromes von Isobutylen 101,8g (0,4 Mol) 2,2,4,4-Tetrachlorbuttersäurechlorid zugetropft. Anschliessend wird während 3 Stunden weitergerührt. Nach der Aufarbeitung analog Beispiel 3 erhält man 69,7g eines nicht kristallisierenden Oels. Die Destillation bei 0,2 mm Hg liefert zwischen 86 und 91°C eine klare, leichtbewegliche Flüssigkeit, die als 2-Chlor-2-(2',2'-dichloräthyl)-3,3-dimethylcyclobutanon identifiziert wird.

IR-Spektrum ($CHCl_3$) in $cm^{-1}$: 1795 (CO).

NMR-Spektrum (100 MHz, $CDCl_3$) in ppm: 1,39 (s, 3H, $CH_3$); 1,42 (s, 3H, $CH_3$); 2,80-3,20 (m, 4H, $2xCH_2$); 6,03 (t, J=6Hz, 1H, C<u>H</u>$Cl_2$).

5 g (21,7 mMol) 2-Chlor-2-(2',2'-dichloräthyl)-3,3-dimethyl-cyclobutanon und 1,7 g Tetrabutylammoniumchlorid werden zusammen 1,5 Stunden bei 125°C gerührt. Die abgekühlte Schmelze wird in Diäthyläther aufgenommen und über Kieselgel filtriert. Das eingedampfte Filtrat (4,5 g) wird über Kieselgel chromatographiert (Eluierung mit Cyclohexan/Toluol in einem 1:1 Volumengemisch). Die Hauptfraktion (farbloses Öl) wird als 2-(2',2'-Dichloräthyl)-3,3-dimethyl-4-chlorcyclobutanon identifiziert.

IR-Spektrum ($CHCl_3$) in $cm^{-1}$: 1795 (CO).

$^1$H-NMR-Spektrum (100 MHz, $CDCl_3$) in ppm : 1,07 (s, 3H, $CH_3$); 1,57 (s, 3H, $CH_3$); 2,15-3,40 (m, 3H); 4,68 (d, J=2Hz, 1H, H-$C_4$); 5,80-5,95 (m, 1H, $CHCl_2$).

$^{13}$C-NMR-Spektrum ($CDCl_3$) in ppm: 198,7 (s, CO); 71,1 (d, $CHCl_2$); 69,3 (d, C-4); 59,7 (d, C-2); 38,3 (t, $CH_2$); 36,3 (s, C-3); 27,6 und 18,7 (je q, je $CH_3$).

4,2 g (18,3 mMol) 2-(2',2'-Dichloräthyl)-3,3-dimethyl-4-chlorcyclobutanon werden mit einer Lösung aus 2,16 g (54,9 mMol) NaOH in 50 ml Wasser versetzt und 3 Stunden bei Raumtemperatur verrührt. Die entstandene klare Lösung wird 2 Stunden auf 100°C erwärmt. Das Reaktionsgemisch wird auf übliche Weise auf Säure aufgearbeitet. Man erhält die bekannte 2-(2'-Chlorvinyl)-3,3-dimethylcyclopropancarbonsäure als Isomerengemisch.

IR-Spektrum ($CDCl_3$) in $cm^{-1}$: 1710 (CO).

Beispiel 5

a) 192 g (1 Mol) Trichloressigsäureäthylester, 200 ml Acetonitril und 3 g Kupfer (I)chlorid werden in einem Autoklaven vorgelegt. Nach dem Aufpressen von 130 g (2 Mol) 1,1-Difluoräthylen wird während 8 Stunden auf 160°C erhitzt. Das Reaktionsgemisch wird anschliessend fraktioniert destilliert. Man erhält 4,4-Difluor-2,2,4-trichlorbuttersäureäthylester; Sdp. 89-91°C/20 mm Hg.

IR-Spektrum ($CHCl_3$) in $cm^{-1}$: 1770 (CO).

NMR-Spektrum (60 MHz, $CDCl_3$) in ppm: 1,40 (t, 3H, $CH_3$); 3,47-3,90 (t, 2H, $CH_2$); 4,20-4,57 (q, 2H, $\underline{CH_2}-CH_3$).

Elementaranalyse für $C_6H_7Cl_3F_2O_2$:

| | | |
|---|---|---|
| berechnet | Cl 41,63% | F 14,87% |
| gefunden | Cl 42,5% | F 13,3%. |

b) 76,6 g (0,3 Mol) 4,4-Difluor-2,2,4-trichlorbuttersäureäthylester und 150 ml konzentrierte Salzsäure werden in einem Bombenrohr 10 Stunden auf 130°C erhitzt. Die nicht wässrige Schicht des Reaktionsgemisches wird abgetrennt, mit Wasser gewaschen, in Toluol aufgenommen, über Magnesiumsulfat getrocknet und eingedampft. Der Rückstand wird destilliert. Man erhält 4,4-Difluor-2,2,4-trichlorbuttersäure als schwach gelb gefärbtes Oel; Sdp. 120-122°C/20 mm Hg.

IR-Spektrum ($CHCl_3$) in $cm^{-1}$: 1750 (CO).

c) 24,5 g (0,1 Mol) 4,4-Difluor-2,2,4-trichlorbuttersäure und 60 g (0,5 Mol) Thionylchlorid werden mit 3 Tropfen N,N-Dimethylformamid versetzt und 3 Stunden bei 40-45°C gehalten. Anschliessend wird 6 Stunden am Rückfluss gekocht. Das überschüssige Thionylchlorid wird abgedampft, und der Rückstand wird einer Destillation unterworfen. Man erhält farbloses 4,4-Difluor-2,2,4-trichlorbuttersäurechlorid; Sdp. 105-109°C/18 mm Hg.

Patentansprüche

1. Eine Verbindung der Formel

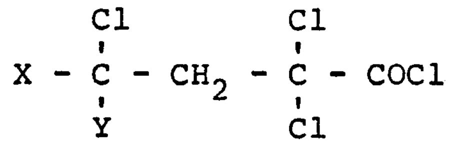

worin X Fluor und Y Wasserstoff oder Fluor oder X Chlor und Y Wasserstoff oder Chlor bedeuten.

2. Die Verbindung gemäss Anspruch 1 der Formel

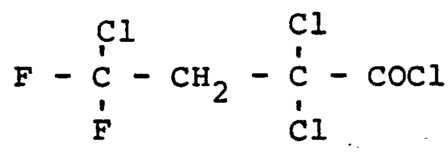

3. Die Verbindung gemäss Anspruch 1 der Formel

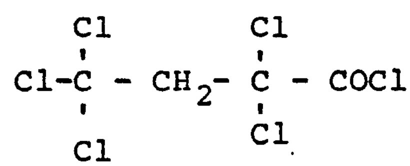

4. Ein Verfahren zur Herstellung einer Verbindung der Formel I nach Anspruch 1, dadurch gekennzeichnet, dass man eine Verbindung der Formel

$CCl_3$-CO-Z

in Gegenwart eines Katalysators und in Gegenwart eines inerten organischen Lösungsmittels an eine Verbindung der Formel

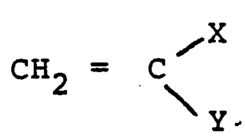

anlager, wobei X und Y die im Anspruch 1 angegebene Bedeutung haben und

Z -Cl, -OH oder -OAlkyl mit 1-4 C-Atomen darstellt, und die Zwischenprodukte der Formel

$$X - \underset{Y}{\overset{Cl}{C}} - CH_2 - \underset{Cl}{\overset{Cl}{C}} - CO- Z$$

worin Z -OH oder -OAlkyl mit 1-4 C-Atomen bedeutet, mit geeigneten Chlorierungsmitteln in Verbindungen gemäss Anspruch 1 überführt.

5. Ein Verfahren nach Anspruch 4, dadurch gekennzeichnet, dass man als Lösungsmittel ein Alkylnitril mit 2 - 5 C-Atomen, besonders Acetonitril, verwendet.

6. Ein Verfahren nach Anspruch 4, dadurch gekennzeichnet, dass man als Lösungsmittel ein 3-Alkoxypropionitril mit 1 oder 2 C-Atomen im Alkoxyteil, besonders 3-Methoxypropionitril, verwendet.

7. Ein Verfahren nach Anspruch 4, dadurch gekennzeichnet, dass man als Katalysator Kupfer(I)chlorid, Kupfer(I)bromid, Kupfer(II)chlorid, Kupfer(II)bromid, Kupferpulver oder Gemische davon verwendet.

0002206

Europäisches Patentamt

# EUROPÄISCHER RECHERCHENBERICHT

Nummer der Anmeldung
EP 78 10 1377

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - C - 835 888 (BASF)<br>* Seite 1, Zeile 24; Seite 2, Zeilen 55-56 *<br>-- | 1,3 |
| A | DE - A - 2 620 101 (PRODUITS CHIMIQUES UGINE KUHLMANN)<br>* Seite 12, Zeilen 5,6 *<br>---- | 1 |

KLASSIFIKATION DER ANMELDUNG (Int.Cl.²)

C 07 C 53/36
51/58//
C 07 C 53/32
53/34-,51/00-,
69/63-,67/30-,
49/27-,45/02-,
61/18-,121/66-,
120/00-,121/75-,
45/00-,27/02

RECHERCHIERTE SACHGEBIETE (Int. Cl.²)

C 07 C 53/00
53/22
53/32
53/34
53/36
51/58

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 31-01-1979 | KLAG |